**Europäisches Patentamt**

**European Patent Office**

**Office européen des brevets**

(11) Numéro de publication: **0 127 905**
**A2**

# (12) DEMANDE DE BREVET EUROPEEN

(21) Numéro de dépôt: **84200213.1**

(22) Date de dépôt: **04.06.81**

(51) Int. Cl.³: **A 61 M 16/00**

(30) Priorité: **10.06.80 FR 8012824**

(43) Date de publication de la demande:
**12.12.84 Bulletin 84/50**

(84) Etats contractants désignés:
**AT BE CH DE FR GB IT LI LU NL SE**

(60) Numéro de publication de la demande initiale
en application de l'article 76 CBE: **0 042 321**

(71) Demandeur: **L'AIR LIQUIDE, SOCIETE ANONYME POUR L'ETUDE ET L'EXPLOITATION DES PROCEDES GEORGES CLAUDE**
**75, Quai d'Orsay**
**F-75321 Paris Cedex 07(FR)**

(72) Inventeur: **Monnier, Jean-Pierre**
**31, avenue du Perche**
**F-78190 Maurepas(FR)**

(74) Mandataire: **Leclercq, Maurice et al,**
**L'AIR LIQUIDE SOCIETE ANONYME POUR L'ETUDE ET L'EXPLOITATION DES PROCEDES GEORGES CLAUDE**
**75, Quai d'Orsay**
**F-75321 Paris Cedex 07(FR)**

(54) **Respirateur à correction automatique de ventilation.**

(57) L'invention concerne un respirateur muni de circuits d'alimentation (10) et d'utilisation (30), d'un accumulateur de gaz respirable (40) d'un distributeur (20) et d'un dispositif de commande à action cyclique (50).

Le respirateur selon l'invention comporte un circuit auxiliaire d'adduction (60) de gaz respirable d'appoint, un circuit d'échappement (70) du gaz respirable et un dispositif de correction de ventilation (80) sensible à la pression dans le circuit d'utilisation et agissant sur les dits circuits d'adduction (60) et d'échappement (70) pour effectuer l'apport et l'évacuation de gaz respirable.

L'invention s'applique aux traitements des insuffisances respiratoires pratiqués en milieu hospitalier ou à domicile.

EP 0 127 905 A2

./...

FIG.3

" RESPIRATEUR A CORRECTION AUTOMATIQUE DE VENTILATION **0127905**

La présente invention concerne un dispositif respirateur pour la ventilation artificielle des voies pulmonaires d'un utilisateur selon un cycle de phases inspiratoires et expiratoires du type comportant :

- un circuit d'alimentation délivrant un gaz respirable ;
- un circuit d'utilisation comportant une branche d'inspiration et une branche d'expiration munie d'une vanne d'expiration ;
- un accumulateur de gaz respirable formant réserve-tampon;
- un distributeur relié audit circuit d'alimentation et comportant une vanne d'inspiration communiquant avec ladite branche d'inspiration, une vanne d'accumulation et une soupape unidirectionnelle communiquant toutes deux avec ledit accumulateur ;
- un dispositif de commande à action cyclique commandant, selon un programme déterminé, les dites vannes d'inspiration, d'expiration et d'accumulation, et prévu pour diriger vers la branche d'inspiration, pendant les phases d'inspiration, le gaz respirable venant du circuit d'alimentation et dudit accumulateur et vers l'accumulateur, pendant les phases d'expiration, le gaz respirable venant dudit circuit d'alimentation.

Ces respirateurs, dans lesquels le gaz respirable est stocké pendant la phase d'expiration puis restitué au circuit d'utilisation pendant la phase d'inspiration, sont destinés principalement au traitement des insuffisances respiratoires et peuvent être utilisés en milieu hospitalier, à domicile, ou encore en secourisme.

Le dispositif de commande à action cyclique permet de délivrer à l'utilisateur une ventilation, c'est-à-dire une quantité donnée de gaz respirable par unité de temps, dont les paramètres, en particulier la fréquence des cycles respiratoires, la durée des temps inspiratoires et expiratoires et le rapport entre ces temps inspiratoires et expiratoires sont affichés et modifiables à volonté.

La ventilation peut donc être facilement contrôlée et réglée par l'opérateur chargé de la conduite du respirateur, généralement un médecin.

Le brevet français n° 76 07.945 du 19 mars 1976, intitulé "Respirateur" décrit un appareil de ce type prévu pour s'adapter automatiquement à la compliance et à la résistance pulmonaire de l'utilisateur.

Des études approfondies effectuées dans le domaine de la ventilation artificielle ont permis de mettre en évidence un certain nombre de difficultés qui se présentent dans la pratique médicale et les insuffisances de certains appareils actuels pour les surmonter. Ces difficultés se rencontrent principalement dans deux cas :

- celui dans lequel la ventilation, réglée par l'opérateur, devient insuffisante pour le patient, et

- celui dans lequel cette ventilation devient excessive pour le patient en raison par exemple d'une obstruction de ses voies pulmonaires au cours du traitement.

La présente invention a pour but de pallier ces difficultés et propose à cet effet un respirateur du type précité mais comportant en outre un circuit auxiliaire d'adduction pour fournir un appoint de gaz respirable au circuit d'utilisation, un circuit d'échappement pour soustraire une partie du gaz respirable audit circuit d'utilisation et un dispositif de correction de ventilation sensible aux variations de pression dans la branche d'inspiration du circuit d'utilisation et à la durée des temps inspiratoire et expiratoire, ledit dispositif de correction commandant lesdits circuits d'adduction et d'échappement pour effectuer un apport ou une évacuation de gaz respirable respectivement, en fonction desdites variations et de ladite durée.

Le respirateur selon l'invention permet donc d'augmenter ou de diminuer la ventilation, de façon automatique, en fonction de la pression dans le circuit d'utilisation, laquelle est tributaire du patient, c'est-à-dire de ses réactions ou de son état.

Selon une autre caractéristique de l'invention, le circuit auxiliaire d'adduction précité comporte un conduit reliant le circuit d'alimentation à l'accumulateur et muni d'une électrovanne et d'un orifice calibré.

Ainsi l'apport supplémentaire de gaz respirable au circuit d'utilisation se fait par l'intermédiaire de l'accumulateur avec un débit dont la valeur est fonction de l'orifice calibré et du temps d'ouverture de l'électrovanne.

Selon encore une autre caractéristique de l'invention, le circuit d'échappement précité comporte une canalisation de mise à l'atmosphère communiquant avec l'accumulateur et munie d'une électrovanne.

Ainsi l'évacuation d'air respirable en excès se fait à partir de l'accumulateur, de façon contrôlée grâce à l'électro-vanne.

D'autres caractéristiques et avantages de l'invention apparaîtront au cours de la description qui va suivre.

Dans les dessins annexés donnés uniquement à titre d'exemple :

- la figure 1 est un diagramme montrant la valeur, en fonction du temps $t$, de la pression $p$ dans le circuit d'utilisation d'un respirateur de type connu dans le cas où la ventilation délivrée par ledit respirateur devient insuffisante.

- la figure 2 est un diagramme identique à celui de la figure 1 mais relatif aux cas où la ventilation délivrée devient excessive.

- la figure 3 représente de façon schématique, un respirateur selon l'invention.

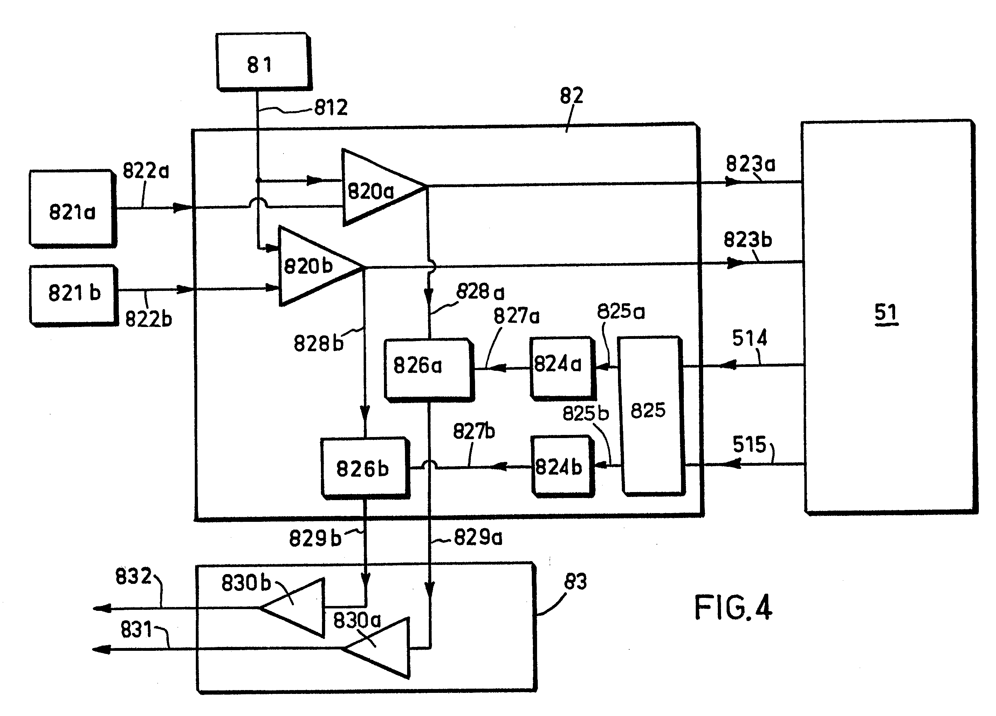

- la figure 4 représente, à plus grande échelle et de façon plus détaillée, le dispositif de correction de ventilation.

- la figure 5 est un diagramme identique à celui de la figure 1 mais obtenu avec le respirateur selon l'invention.

- la figure 6 est un diagramme identique à celui de la figure 2 mais obtenu avec le respirateur selon l'invention.

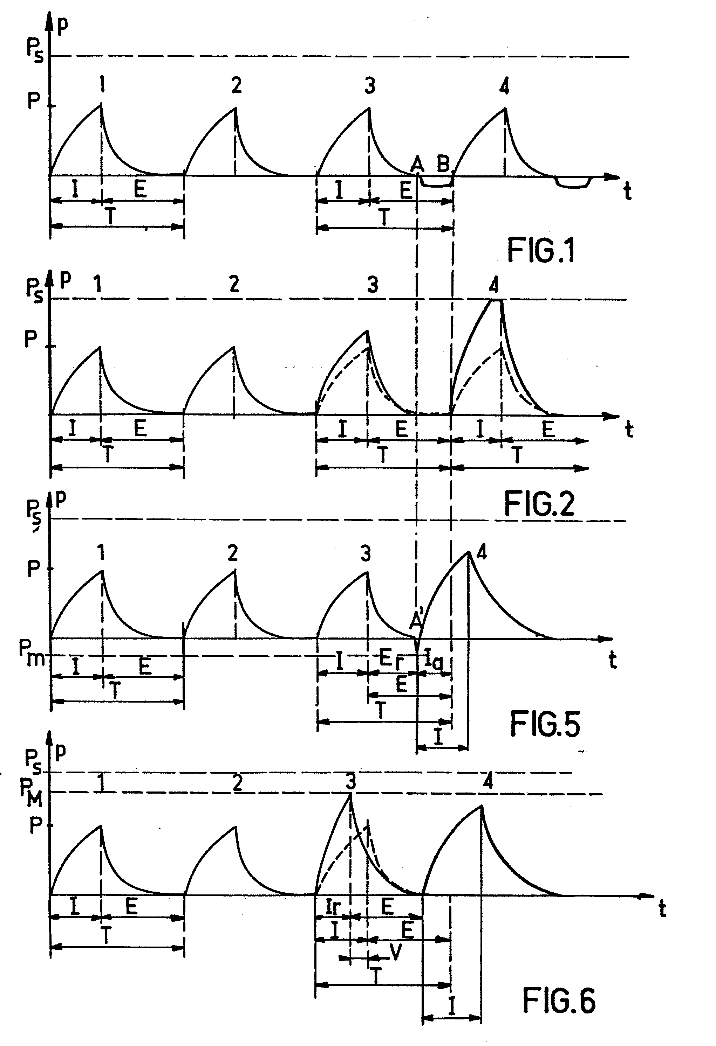

En se référant à la figure 1, on voit que la pression dans le circuit d'utilisation, ou circuit patient, croît pendant la durée I de la phase d'inspiration, ou temps inspiratoire, depuis une valeur prise pour origine (pression pulmonaire résiduelle) jusqu'à une valeur maximale P puis décroît pendant la durée E de la phase expiratoire, ou temps expiratoire, jusqu'à la valeur d'origine, la somme de ces durées étant la période respiratoire T (I + E = T). Il est à noter que la pression dans le circuit d'utilisation ne peut en aucun cas dépasser une valeur de sécurité Ps, ceci grâce à une soupape de sécurité prévue dans ledit circuit. Les valeurs I, E, T, ainsi que la quantité de gaz insufflée, sont réglées par l'opérateur de façon à ce qu'elles soient conformes aux besoins et aux caractéristiques du patient. La valeur de P résulte de ce réglage. Les cycles respiratoires correspondants sont représentés en 1 et 2.

Si la ventilation devient insuffisante par suite de modifications des caractéristiques du patient, celui-ci réagit par un effort inspiratoire pendant la phase expiratoire, c'est-à-dire entre le moment où il a fini d'expirer (A) et le début de la phase inspiratoire suivante (B), imposée par le respirateur. Cet effort inspiratoire crée dans le circuit patient, entre A et B, une dépression qui traduit l'insuffisance de ventilation (cycles 3 et 4).

La figure 2 dans laquelle les mêmes lettres désignent les mêmes paramètres que dans la figure 1, est relative au cas d'un patient dont les voies pulmonaires sont obstruées. Dans un premier temps (cycle 3), la pression s'élève fortement dans le circuit patient. Ceci se traduit par une augmentation du niveau d'énergie dans l'accumulateur et une augmentation de la pression qui se poursuit à chaque cycle jusqu'à atteindre la valeur de la pression de sécurité Ps (cycle 4). La ventilation délivrée par le respirateur est trop importante.

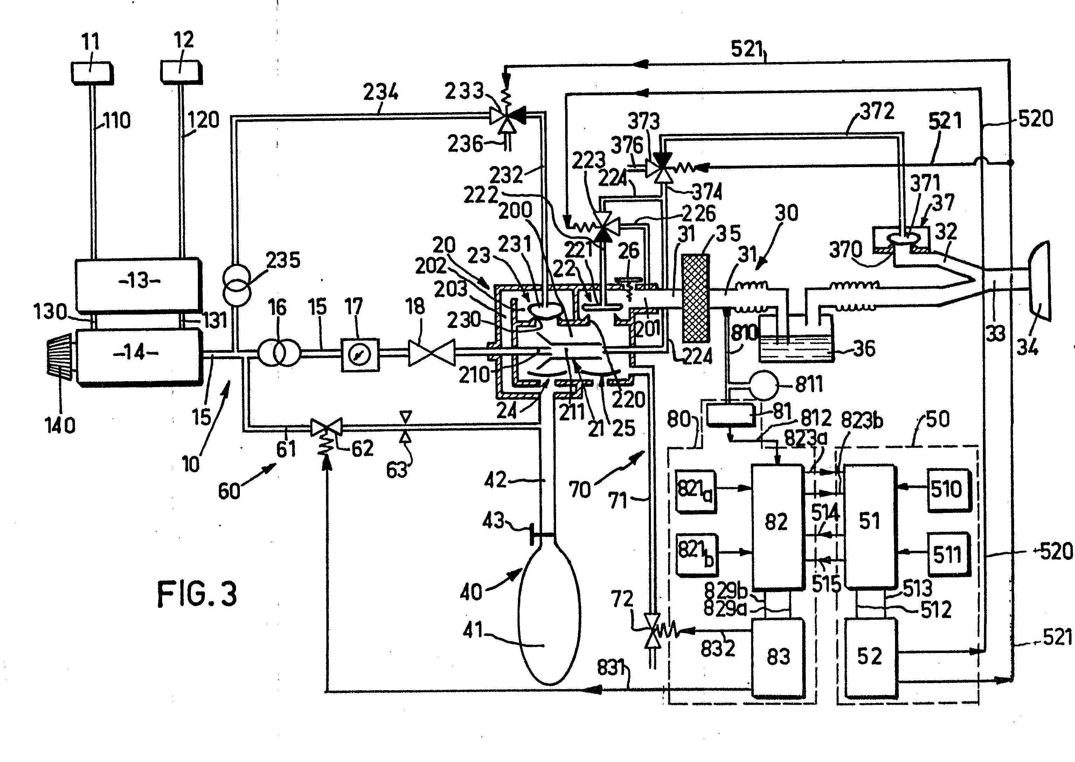

Selon le mode de réalisation représenté à la figure 3, le respirateur selon l'invention comporte essentiellement un circuit d'alimentation en gaz respirable 10, un bloc distributeur 20, un circuit d'utilisation ou circuit patient 30, un accumulateur pneumatique 40, ce circuit 30 et cet accumulateur recevant le gaz respirable du circuit 10 par l'intermédiaire du distributeur 20, et un dispositif de commande électrique à action cyclique (50), dont la fonction est de contrôler les cycles successifs d'inspiration et d'expiration et d'assurer la respiration du patient.

Le circuit d'alimentation 10 comporte deux sources 11 et 12 de gaz sous pression, par exemple, dans le cas où le patient doit être ventilé en air suroxygéné, une source d'air et une source d'oxygène.

Les sources 11 et 12 communiquent, par des conduits 110 et 120, avec un égalisateur de pression 13 qui communique lui-même, par des conduits 130 et 131, avec un mélangeur 14 muni d'un bouton de réglage 140 qui permet le dosage du mélange des deux gaz.

Le mélange 14 communique avec une canalisation 15 munie d'un détendeur 16 qui ramène la pression du gaz à une valeur stable, de l'ordre de 1 bar, d'un débitmètre 17 et d'un robinet de réglage de débit 18.

Le distributeur 20 comporte un venturi 21 dont l'injecteur 210, relié à la canalisation 15, reçoit le gaz respirable et dont le divergent 211 débouche directement dans un espace interne 200 du distributeur. Ledit espace 200 communique avec le circuit d'utilisation 30 par une vanne d'inspiration 22 logée dans un compartiment 201, avec l'accumulateur 40 d'une part par l'intermédiaire d'une vanne d'accumulation 23 logée dans un compartiment 202 communiquant avec un passage 203 et d'autre part par une soupape unidirectionnelle 24 (laissant passer le gaz dans le sens allant de l'accumulateur 40 vers le circuit 30) et enfin avec l'atmosphère avec une soupape d'air additionnel 25. Le compartiment 201 communique avec l'atmosphère par une soupape tarée 26 qui empêche la pression dans le circuit 30 de dépasser la valeur de sécurité Ps.

La vanne d'inspiration 22 comporte un siège 220, un clapet pneumatique 221 relié, par un conduit 222, à une électrovanne 223 qui a pour fonction le gonflage et le dégonflage dudit clapet et communique à cet effet, d'une part, par un conduit 224, avec la sortie du divergent 211 du venturi 21 et d'autre part, par un conduit 226, avec le compartiment 201 du distributeur 20.

La vanne d'accumulation 23 comporte un siège 230, un clapet pneumatique 231 qui communique, par l'intermédiaire d'un conduit 232, avec une électrovanne 233 de gonflage et de dégonflage dudit clapet. L'électrovanne 233 communique d'une part avec la canalisation 15, par l'intermédiaire d'un conduit 234 muni d'un détendeur 235, et d'autre part avec l'atmosphère par une purge 236.

Le circuit d'utilisation 30 comporte une branche d'inspiration 31 qui communique directement avec le compartiment 201 du distributeur 20 et une branche d'expiration 21, ces deux branches 31 et 32 communiquant, par l'intermédiaire d'un tronc commun 33 avec un masque 34 pour le patient. La branche 31 comporte un filtre bactériologique 35 et un humidificateur 36. La branche d'expiration 32 communique avec l'atmosphère par une vanne d'expiration 37. La vanne d'expiration 37 comporte un siège 370, un clapet pneumatique 371 qui communique, par un conduit 372, avec une électrovanne 373 de gonflage et de dégonflage dudit clapet. L'électrovanne 373 est reliée d'une part à la sortie du divergent 211 par l'intermédiaire d'un conduit 374 et du conduit précité 224 de la vanne d'inspiration et d'autre part avec l'atmosphère par une purge 376.

L'accumulateur 40 est constitué d'un ballon élastique 41, dit ballon intégrateur, prévu pour stocker une partie de gaz respirable formant ainsi une réserve tampon et qui est relié au distributeur 20 par une tubulure 42 munie d'une soupape de sécurité 43.

Le dispositif de commande 50 comporte essentiellement une horloge électronique 51 et un organe de contrôle 52 constitué par exemple par un amplificateur de puissance et piloté par ladite horloge par l'intermédiaire des conducteurs 512 et 513. Le dispositif 50 est prévu pour commander, selon un programme déterminé, la fréquence des cycles respiratoires $\frac{1}{I+E}$ et le rapport I/E du temps inspiratoire sur le temps expiratoire pour chaque cycle respiratoire, cette fréquence et ce rapport étant réglables au moyen d'organes de réglage 510 et 511 respectivement, constitués par exemple par des potentiomètres et associés à l'horloge 51. L'organe de contrôle 52 est relié, par un conducteur 520, à l'électrovanne 223 et par un conducteur commun 521 aux électrovannes 233 et 373 et il est prévu pour envoyer aux dites électrovannes, en fonction des signaux de pilotage qu'il reçoit de l'horloge 51, des signaux qui commandent leur ouverture et leur fermeture et par conséquent l'ouverture et la fermeture des vannes pneumatiques 22, 23 et 37.

L'opérateur peut donc imposer au patient une suite de temps inspiratoires et expiratoires déterminés.

Le respirateur selon l'invention comporte, outre les constituants sus-mentionnés, un circuit auxiliaire d'adduction 60, un circuit d'échappement 70 et un dispositif de correction de ventilation 80.

Le circuit auxiliaire d'adduction 60 comporte essentiellement un conduit 61 muni d'une électrovanne 62 qui fonctionne par tout ou rien et d'un orifice calibré 63. Le conduit 61 relie directement la canalisation 15 du circuit d'alimentation 10 à la tubulure 42 de l'accumulateur 40.

Le circuit d'échappement 70 comporte une canalisation 71 de mise à l'atmosphère reliée à l'espace interne 200 du distributeur 20 et munie, à son extrémité, d'une électrovanne 72 qui fonctionne par tout ou rien.

Le dispositif de correction de ventilation 80 comporte un capteur de pression 81, un calculateur électronique 82 sensible aux indications dudit capteur et un organe de contrôle 83, piloté par ledit calculateur.

Le capteur 81, par exemple du type piezo-résistif, est relié par un conduit 810 muni d'un manomètre 811, à la branche d'inspiration 31, il détecte la pression dans ladite branche et délivre au calculateur 82, par l'interméidiaire du conducteur électrique 812, une tension électrique proportionnelle à ladite pression.

Le calculateur 82 est prévu pour commander l'horloge 51 de façon qu'elle déclenche, lorsque la pression dans la branche 31 atteint une valeur minimale $P_m$ déterminée (dépression) ou une valeur maximale $P_M$ déterminée (inférieure ou au plus égale à la pression de sécurité $P_s$), une phase inspiratoire ou une phase expiratoire anticipée, c'est-à-dire en avance sur la phase inspiratoire ou expiratoire qui se serait normalement produite selon le programme déterminé par les organes de réglage 510 et 511. Les organes 510 et 511 déterminent en effet la fréquence $\dfrac{1}{I + E}$ des cycles respiratoires ainsi que le rapport I/E du temps inspiratoire sur le temps expiratoire, ils déterminent par conséquent I et E. Le fait de déclencher par anticipation une phase inspiratoire ou une phase expiratoire a pour conséquence de raccourcir le temps expiratoire ou le temps inspiratoire du cycle respiratoire programmé, ces deux temps prenant alors des valeurs Er et Ir, inférieures a E et I respectivement.

Le calculateur 82 est prévu également pour mettre en mémoire les temps I et E et pour déterminer les différences E − Er et I − Ir de façon à commnader la durée d'ouverture des vannes 62 et 72 en fonction de ces différences de temps E − Er et I − Ir.

Le calculateur 82 comporte deux circuits comparateurs 820 a et 820 b pour comparer les tensions électriques délivrées par le capteur 81 à deux tensions de référence, l'une minimale, correspondant à la pression minimale $P_m$, l'autre maximale, correspondant à la pression maximale $P_M$. Ces deux tensions de référence, et par conséquent les deux pressions $P_m$ et $P_M$, peuvent être choisies à volonté par l'opérateur (avec la condition $P_M < P_S$) au moyen des organes de réglage 821 a et 821 b, constitués par exemple par des potentiomètres, et reliés, par des conducteurs 822 a et 822 b, à l'une des entrées de chacun des comparateurs. Les comparateurs 820a et 820 b ont leur autre entrée reliée à la sortie du capteur 81 par un conducteur 812 et leur sortie reliée, par des conducteurs 823 a et 823 b, à la base de temps (non représentée) de l'horloge 51.

Le calculateur 82 comporte également deux circuits de mémoire 824 a et 824 b et deux circuits soustracteurs 826 a et 826 b. Les circuits 824 a et 824 b reçoivent, à leur entrée, les temps I et E programmés par l'horloge 51 et les mettent en mémoire.

Ces temps I et E sont calculés par un organe de calcul 825, relié par des conducteurs 514 et 515, aux circuits (non représentés) de l'horloge 51 qui délivrent les valeurs $\dfrac{1}{I + E}$ et $\dfrac{I}{E}$ et sont adressés aux mémoires 824 a et 824 b par les conducteurs 825 a et 825 b. Les soustracteurs 826 a et 826 b reçoivent d'une part les temps I et E des mémoires 824 a et 824 b par l'intermédiaire des conducteurs 827 a et 827 b et d'autre part les temps Ir et Er des comparateurs 820 a et 820 b par l'intermédiaire des conducteurs 828 a et 828 b. Les différences I - Ir et E - Er sont adressées, par des conducteurs 829 a et 829 b, à l'organe de contrôle 83.

L'organe de contrôle 83 comporte deux amplificateurs 830a et 830b dont les entrée sont reliées aux soustracteurs 826a et 826b par les conducteurs précités 829a et 829b, leurs sorties étant reliées aux électrovannes 62 et 72 par l'intermédiaire des conducteurs 831 et 832 respectivement, pour commander l'ouverture et la fermeture des dites électrovannes en fonction de signaux qu'ils reçoivent des soustracteurs 826 a et 826 b.

Le fonctionnement du distributeur selon l'invention est le suivant :

Les vannes 22, 23 et 37, commandées par le dispositif de commande 50, provoquent selon qu'elles sont ouvertes ou fermées, un cycle d'inspiration ou au contraire un cycle d'expiration. Pendant l'inspiration (cas représenté à la figure 3) la vanne 22 est ouverte et les vannes 23 et 37 fermées. Le gaz respirable venant du circuit d'alimentation 10 par le venturi 21 et celui venant du ballon 41 par l'intermédiaire de la soupape unidirectionnelle 24 passent de l'espace interne 200 dans le compartiment 201 du distributeur 20, puis dans la branche 31 du circuit d'utilisation 30. Pendant l'expiration, la vanne 22 est fermée et les vannes 23 et 37 ouvertes. Le gaz expiré par le patient passe dans l'atmosphère par la branche d'expiration 32 et la vanne 37 tandis que le gaz respirable venant du circuit d'alimentation 10 par le venturi 21 passe de l'espace interne 200 au ballon 41 par l'intermédiaire du compartiment 202, du passage 203 et de la tubulure 42. Si la ventilation ainsi délivrée au patient est compatible en fréquence et en quantité avec les besoins et les caractéristiques pulmonaires du patient, c'est-à-dire si cette ventilation se fait selon les cycles 1 et 2 des figures 5 et 6, les électrovannes 62 et 72 associées au circuit d'adduction 60 et au circuit d'échappement 70 sont maintenues fermées par le dispositif de correction 80.

Si la ventilation devenant insuffisante, le patient fait un effort inspiratoire, il s'ensuit dans la branche 31 du circuit d'utilisation une dépression qui apparaît en A' sur la figure 5 (correspondant au point A de la figure 1). Cette dépression, détectée par le capteur 81, est convertie en une tension électrique proportionnelle qui est reçue par les comparateurs 820 a et 820 b. Cette tension est comparée aux tensions de référence réglées par les organes de réglage 821 a et 821 b et dès qu'elle atteint la valeur de la tension de référence réglée par 821a et correspondant à Pm, le comparateur 820a envoie un signal électrique à l'horloge 51 par le conducteur 823 a et au soustracteur 826 a, par le conducteur 827a. L'horloge 51 pilote l'organe de contrôle 52 pour qu'il déclenche une phase inspiratoire anticipée. Le temps expiratoire est donc raccourci à une valeur réelle Er inférieure au temps E programmé par le dispositif 50. Le soustracteur 826a détermine la différence entre le temps expiratoire E mis en mémoire qui aurait dû avoir lieu et le temps expiratoire Er qui s'est produit. La différence des deux temps permet au soustracteur 826 a de commander, par l'intermédiaire de l'amplificateur 830a, l'ouverture de l'électrovanne 62 pendant une durée E - Er = Ia et d'alimenter le ballon 41 en gaz respirable d'appoint. Le niveau d'énergie dans le ballon intégrateur 41 augmente, ce qui entraîne une augmentation du débit d'inspiration. La ventilation se fait alors selon les cycles 3 et 4 de la figure 5. Le cycle 4 empiète sur le cycle 3 et sa pression de pointe dépasse la valeur P des cycles précédents.

Si, au contraire, la ventilation devient excessive par suite d'une obstruction des voies pulmonaires du patient, il s'ensuit une augmentation de pression dans le circuit d'utilisation. Cette pression détectée par le capteur 81 est convertie en tension électrique proportionnelle qui est reçue par les comparateurs 820a, 820b. Dès que cette tension atteint la valeur de référence réglée par 821 b et correspondant à $P_M$, le comparateur 820 b envoie un signal électrique à l'horloge 51 par le conducteur 823 b et au soustracteur 826 b par le conducteur 827 b. L'horloge 51 pilote l'organe de contrôle 52 pour qu'il déclenche une phase expiratoire anticipée. Le temps inspiratoire est donc raccourci à une valeur réelle Ir inférieure au temps inspiratoire I programmé par le dispositif 50. Le soustracteur 826 b détermine également la différence entre le temps inspiratoire I mis en mémoire qui aurait dû avoir lieu et le temps inspiratoire Ir qui s'est produit. La différence des deux temps permet au soustracteur 826 b, par l'intermédiaire de l'amplificateur 830 b, de commander l'ouverture de l'électrovanne 72 pendant une durée I - Ir = V, de façon à rejeter à l'extérieur une partie du gaz respirable stocké dans le ballon 41, provoquant sa décompression partielle. Le niveau d'énergie dans le ballon 41 diminue et par conséquent le débit d'inspiration diminue également.

Les cycles d'inspiration et d'expiration présentent alors la forme représentée par les cycles 3 et 4 de la figure 6. La pression de pointe du cycle 3 atteint la valeur $P_M$, tandis que la pression de pointe du cycle 4 est ramenée à une valeur inférieure à $P_M$, en raison de la décompression du ballon 41.

On pourrait alors apporter au mode de réalisation décrit et représenté de nombreuses variantes sans pour autant sortir du cadre de l'invention.

0127905

## REVENDICATIONS

1. Respirateur pour la ventilation artificielle des voies pulmonaires d'un utilisateur selon un cycle de phases inspiratoires et expiratoires du type comportant un circuit d'alimentation (10) délivrant en continu un gaz respirable; un circuit d'utilisation (30) comportant une branche d'inspiration (31) et une branche d'expiration (32) munie d'une soupape d'expiration (37); un accumulateur de gaz respirable (40) formant réserve-tampon; un distributeur (20) relié audit circuit d'alimentation (10) et comportant une soupape d'inspiration (22) communiquant avec ladite branche d'inspiration (31), une vanne d'accumulation (23) et une soupape unidirectionnelle (24) toutes deux de communication avec l'accumulateur (40) et un dispositif de commande à action cyclique (50) commandant, selon un programme déterminé, les dites soupapes d'inspiration (22), d'expiration (37) et d'accumulation (23) et agencé pour diriger vers la branche d'inspiration (31) pendant les phases d'inspiration le gaz respirable venant du circuit d'alimentation (10) et de l'accumulateur (40), pendant les phases d'expiration le gaz respirable venant du circuit d'alimentation (10) vers l'accumulateur (40), caractérisé par :

- un circuit d'échappement (70) à vanne (72) de l'accumulateur (40) vers l'atmosphère ;

- un dispositif de correction de ventilation (80) sensible aux variations de la pression dans la branche d'inspiration (31) de correction de la durée du temps inspiratoire et du début du temps expiratoire suivant avec action sur le circuit d'échappement (70) pour effectuer une évacuation d'air respirable en cas d'élévation anormale de pression dans le circuit inspiratoire (31)

2. Respirateur selon la revendication 1, caractérisé en ce que la vanne (72) du circuit d'échappement (70) est commandée à ouverture pendant un laps de temps (V) s'écoulant entre l'instant (IR) où la pression dans le circuit d'inspiration (31) atteint la pression maximum limite (PM) et l'instant théorique de fin d'inspiration (I).

3. Respirateur selon la revendication 1, caractérisé en ce que la durée de la phase expiratoire qui suit une phase inspiratoire de durée réduite (IR) est égale à la durée (E) d'une phase expiratoire théorique.

4. Respirateur selon la revendication 1, caractérisé en ce que la canalisation (71) communique avec l'accumulateur (40) respectivement en aval de la soupape unidirectionnelle (24) pour le sens d'écoulement du gaz dudit accumulateur (40) vers ladite branche d'inspiration (31).

5. Respirateur selon la revendication 1 ou 2, caractérisé en ce que le dispositif de correction de ventilation (80) précité comporte un capteur de pression (81) prévu pour délivrer des signaux électriques en fonction de la pression dans la branche d'inspiration (31), un calculateur électronique (82) relié audit capteur ainsi qu'au dispositif de commande à action cyclique (50) et un organe de contrôle (83) piloté par ledit calculateur et prévu pour commander l'ouverture et la fermeture de l'électrovanne (72).

6. Respirateur selon la revendication 5, caractérisé en ce que le capteur de pression (81) précité est du type piezo-résistif et prévu pour délivrer une tension proportionnelle-à la pression qu'il reçoit de la branche d'inspiration (31).

7. Respirateur selon la revendication 6, caractérisé en ce que le calculateur électronique (82) comporte un circuit comparateur (820b) ayant l'une de leurs entrées reliée au capteur (81) et leur autre entrée reliée à un organe de réglage (821b) prévus pour délivrer une tension de référence, maximale, lesdits comparateurs pilotant l'organe de commande à action cyclique (50) pour qu'il déclenche une phase expiratoire lorsque la tension électrique délivrée par ledit capteur (81) atteint la valeur maximale précitée.

8. Respirateur selon la revendication 7, caractérisé en ce que le calculateur (82) comporte des circuits de mémoire (824a, 824b) pour la mise en mémoire des temps inspiratoire I et expiratoire E programmés par l'organe de commande (50) et un circuit soustracteur (826b) ayant l'une de ses entrées reliée à ladite mémoire (824b) et son autre entrée reliée au comparateur (820b) qui délivre des temps inspiratoire Ir et expiratoire Er inférieurs à I et E et résultant du déclenchement de l'organe de commande (50) en fonction de la valeur de référence maximale précitée, les dits

**0127905**

soustracteurs (826b) pilotant l'organe de contrôle (83) pour qu'il commande la durée d'ouverture de l'électrovanne (72) en fonction de la différence $I - I_r = V$.

9. Respirateur selon la revendication 8, caractérisé en ce que l'organe de contrôle (83) comporte un circuit amplificateur (830b) ayant ses entrées reliées au soustracteur précité (826b) et sa sortie reliée à l'électrovanne (72) précitée.

0127905

1/3

FIG.1

FIG.2

FIG.5

FIG.6

FIG.3

2/3

0127905

0127905

FIG.4